(19) Europäisches Patentamt
European Patent Office
Office européen des brevets

(11) Veröffentlichungsnummer: **0 346 789 B1**

(12) **EUROPÄISCHE PATENTSCHRIFT**

(45) Veröffentlichungstag der Patentschrift: **20.04.94**

(51) Int. Cl.5: **C07D 239/60**, C07D 403/12, C07D 251/30, C07D 251/38, A01N 43/54, A01N 43/66

(21) Anmeldenummer: **89110566.0**

(22) Anmeldetag: **10.06.89**

Die Akte enthält technische Angaben, die nach dem Eingang der Anmeldung eingereicht wurden und die nicht in dieser Patentschrift enthalten sind.

(54) **Salicylsäurederivate und deren Schwefelanaloge.**

(30) Priorität: **16.06.88 DE 3820484**
**04.02.89 DE 3903365**

(43) Veröffentlichungstag der Anmeldung:
**20.12.89 Patentblatt 89/51**

(45) Bekanntmachung des Hinweises auf die Patenterteilung:
**20.04.94 Patentblatt 94/16**

(84) Benannte Vertragsstaaten:
**AT BE CH DE ES FR GB IT LI NL SE**

(56) Entgegenhaltungen:
**EP-A- 0 249 708**
**EP-A- 0 287 079**
**EP-A- 0 315 889**
**EP-A- 0 336 494**

(73) Patentinhaber: **BASF Aktiengesellschaft**
**Carl-Bosch-Strasse 38**
**D-67063 Ludwigshafen(DE)**

(72) Erfinder: **Rheinheimer, Joachim, Dr.**
**Merziger Strasse 24**
**D-6700 Ludwigshafen(DE)**
Erfinder: **Eicken, Karl, Dr.**
**Am Huettenwingert 12**
**D-6706 Wachenheim(DE)**
Erfinder: **Plath, Peter, Dr.**
**Hans-Balcke-Strasse 13**
**D-6710 Frankenthal(DE)**
Erfinder: **Paul, Gerhard, Dr.**
**Berner Weg 34**
**D-6700 Ludwigshafen(DE)**
Erfinder: **Harreus, Albrecht, Dr.**
**Teichgasse 13**
**D-6700 Ludwigshafen(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**
Erfinder: **Grossmann, Klaus, Dr.**
**Wilhelm-Busch-Strasse 5**
**D-6703 Limburgerhof(DE)**
Erfinder: **Rademacher, Wilhelm, Dr.**
**Austrasse 1**
**D-6703 Limburgerhof(DE)**
Erfinder: **Jung, Johann, Prof. Dr.**
**Hardenburgstrasse 19**
**D-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft Salicylsäurederivate und deren Schwefelanaloge der Formel I,

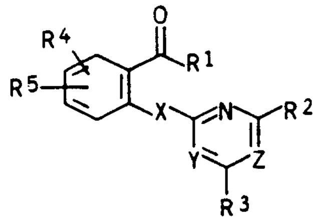

I

in der die Substituenten folgende Bedeutung haben:

- $R^1$ eine Succinyliminooxygruppe;
  ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
  einen Rest -$OR^6$, worin
- $R^6$ Wasserstoff, ein Alkalimetallkation, das Äquivalent eines Erdalkalimetallkations oder ein organisches Ammoniumion;
  eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, oder
  eine $C_3$-$C_6$-Alkenyl- oder eine $C_3$-$C_6$-Alkinylgruppe, wobei diese Gruppen ihrerseits ein bis fünf Halogenatome tragen können;
  bedeutet, oder
  einen Rest $ON=CR^7R^8$, worin
- $R^7$ und $R^8$ $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann
  bedeutet;
- $R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
- X ein Sauerstoff- oder Schwefelatom;
- Y,Z ein Stickstoffatom oder eine Methingruppe =CH-;
- $R^4$ ein Halogenatom,
  eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio;
  eine $C_3$-$C_6$-Alkenyl-, $C_3$-$C_6$-Alkenyloxy-, $C_3$-$C_6$-Alkinyl- oder $C_3$-$C_6$--Alkinyloxygruppe, welche ein bis fünf Halogenatome und/oder einen der folgenden Reste tragen kann: $C_1$-$C_4$-Alkoxy oder $C_1$-$C_4$-Alkylthio;
  eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe;
- $R^5$ Wasserstoff oder einen der Reste $R^4$

mit der Maßgabe, daß $R^2$ eine $C_1$-$C_4$-Halogenalkylgruppe oder Y eine Methingruppe =CH- oder $R^4$ eine $C_3$-$C_6$-Alkenylgruppe, welche ein bis fünf Halogenatome tragen kann oder eine $C_3$-$C_6$-Halogenalkenyloxygruppe bedeutet, wenn $R^1$ für -$OR^6$ und X für Sauerstoff steht, sowie ferner mit der Maßgabe, daß wenn $R^1$ für $OR^6$ und X Schwefel steht, $R^6$ eine $C_3$-$C_6$-Alkenyl- oder $C_3$-$C_6$-Alkinylgruppe darstellt, wobei die Gruppen 1 bis 5 Halogenatome tragen können, sowie deren umweltverträglichen Salze.

Weiterhin betrifft die Erfindung Verfahren zur Herstellung der Verbindungen I sowie ihre Verwendung als Herbizide sowie Wachstumsregulatoren der allgemeinen Formel Ia,

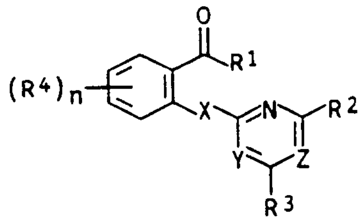

Ia

in der die Substituenten folgende Bedeutung haben:

$R^1$ eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest -$OR^6$, worin

$R^6$ Wasserstoff, ein Alkalimetallkation, das Äquivalent eines Erdalkalimetallkations oder ein organisches Ammoniumion;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, oder
eine $C_3$-$C_6$-Alkenyl- oder eine $C_3$-$C_6$-Alkinylgruppe, wobei diese Gruppen ihrerseits ein bis fünf Halogenatome tragen können;
bedeutet, oder
einen Rest $ON=CR^7R^8$, worin

$R^7$ und $R^8$ $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann bedeutet;

$R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

X ein Sauerstoffatom;

Y ein Stickstoffatom;

Z ein Stickstoffatom oder eine Methingruppe =CH-;

$R^4$ ein Halogenatom,
eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio;
eine $C_3$-$C_6$-Alkenyl-, $C_3$-$C_6$-Alkenyloxy-, $C_3$-$C_6$-Alkinyl- oder $C_3$-$C_6$-Alkinyloxygruppe, welche ein bis fünf Halogenatome und/oder einen der folgenden Reste tragen kann: $C_1$-$C_4$-Alkoxy oder $C_1$-$C_4$-Alkylthio;
eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe und

n 0, 1 oder 2.

In der Literatur (EP-A 223 406, EP-A 249 707, EP-A 249 708, EP-A 287 072 und EP-A 287 079) sind herbizid wirksame substituierte Salicylsäuren und deren Schwefelanaloge beschrieben. Ihre Wirkung ist jedoch unbefriedigend.

Das nachveröffentlichte Dokument EP-A-315 889 beschreibt herbizid wirksame [(4,6-Dimethoxypyrimidin-2-yl)-thio]benzoesäurederivate mit Halogen, Alkyl- oder Phenoxysubstitution im Phenylkern.

Aufgabe der vorliegenden Erfindung waren daher neue Salicylsäurederivate bzw. deren Schwefelanaloge mit verbesserten herbiziden Eigenschaften sowie mit pflanzenwachstumsregulierenden Eigenschaften.

Entsprechend dieser Aufgabe wurden die eingangs definierten Verbindungen der Formel I gefunden. Außerdem wurden Verfahren zur Herstellung der Verbindungen I und Verfahren zur Bekämpfung unerwünschten Pflanzenwuchses mit den Verbindungen I gefunden. Es wurde außerdem gefunden, daß Salicylsäurederivate der vorstehend definierten allgemeinen Formel Ia ausgezeichnete pflanzenwachstumsregulierende Eigenschaften besitzen.

Verbindungen der Formel I erhält man beispielsweise, indem man ein entsprechend substituiertes Benzoesäurederivat der Formel II, das bekannt ist oder nach üblichen Methoden ausgehend von bekannten

Vorprodukten hergestellt werden kann, mit einer entsprechenden Verbindung der Formel III in Gegenwart einer Base umsetzt.

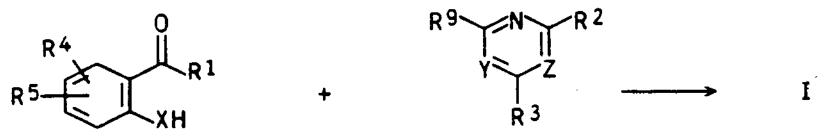

II III

$R^9$ in Formel III bedeutet Halogen wie Chlor, Brom und Iod, Aryl- oder Alkylsulfonyl wie Toluolsulfonyl und Methylsulfonyl oder eine andere äquivalente Abgangsgruppe. Verbindungen der Formel III mit einem reaktionsfähigen Substituenten $R^9$ sind bekannt oder mit dem allgemeinen Fachwissen leicht zu erhalten. Als Base können Alkali- oder Erdalkalimetallhydride wie NaH und $CaH_2$, Alkalimetallhydroxide wie NaOH und KOH, Alkalimetallcarbonate wie $Na_2CO_3$ und $K_2CO_3$, Alkalimetallamide wie $NaNH_2$ und Lithiumdiisopropylamid oder tertiäre Amine Verwendung finden. Bei Einsatz einer anorganischen Base kann man einen Phasentransferkatalysator zusetzen, wenn dies den Umsatz fördert.

Soweit es sich bei den in der beschriebenen Weise hergestellten Verbindungen der Formel I um Carbonsäuren handelt (wenn also $R^1$ Hydroxyl bedeutet), können hieraus andere beschriebene Verbindungen z.B. auch dadurch hergestellt werden, daß man die Carbonsäure zunächst auf übliche Weise in eine aktivierte Form wie ein Halogenid oder Imidazolid überführt und dieses dann mit der entsprechenden Hydroxylverbindung umsetzt. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Im Hinblick auf die herbizide Wirksamkeit sind Verbindungen I bevorzugt, in denen die Substituenten folgende Bedeutung haben:

$R^1$ Succinyliminooxy,

5-gliedriges Heteroaryl wie Pyrrolyl, Pyrazolyl, Imidazolyl und Triazolyl, insbesondere Imidazolyl, Triazolyl und Pyrazolyl, wobei der aromatische Rest über Stickstoff gebunden ist und seinerseits ein bis vier Halogenatome wie vorstehend genannt, insbesondere Fluor und Chlor und/oder ein bis zwei der folgenden Reste tragen kann: Alkyl, wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl; Alkoxy wie vorstehend genannt, mit ein bis vier Kohlenstoffatomen wie insbesondere Methoxy, Ethoxy, Propyloxy, 1-Methylethoxy, Butyloxy, 1-Methylpropyloxy, 2-Methylpropyloxy, 1,1-Dimethylethoxy, Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und/oder

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;

ein Rest $OR^6$, worin

$R^6$ Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion;

$C_1$-$C_{10}$-Alkyl wie insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, welches ein bis fünf der vorstehend genannten Halogenatome, insbesondere Fluor und Chlor und/oder einen der folgenden Reste tragen kann: Cyano, Alkoxy

bzw. Alkylthio mit ein bis vier Kohlenstoffatomen wie vorstehend genannt, insbesondere Methoxy, Ethoxy, 1-Methylethoxy und Methylthio;

$C_1$-$C_8$-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;

$C_1$-$C_8$-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl.

Phenyl, Phenoxy oder Phenylcarbonyl, wobei diese aromatischen Reste ihrerseits ein bis fünf Halogenatome wie vorstehend genannt, insbesondere Fluor, Chlor und Brom und/oder ein bis drei der folgenden Reste tragen können:

Alkyl, Halogenalkyl, Alkoxy, Halogenalkoxy und/oder Alkylthio mit jeweils ein bis vier Kohlenstoffatomen wie im allgemeinen und im besonderen vorstehend genannt, oder

$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;

$C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl, wobei diese Alkenyl- und Alkinylgruppen ein bis fünf der vorstehend im allgemeinen und im besonderen genannten Halogenatome tragen können, bedeutet;

ein Rest $ON=CR^7R^8$, worin

$R^7$, $R^8$ unverzweigtes oder verzweigtes $C_1$-$C_{20}$-Alkyl, vorzugsweise $C_1$-$C_{15}$-Alkyl, insbesondere $C_1$-$C_9$-Alkyl, welches einen Phenylrest tragen kann, Phenyl oder gemeinsam $C_3$-$C_{12}$-Alkylen, vorzugsweise $C_4$-$C_7$-Alkylen, welches ein bis drei $C_1$-$C_3$-Alkylgruppen, vorzugsweise Methyl- oder Ethylgruppen tragen kann, bedeutet;

$R^2$, $R^3$ im allgemeinen und im besonderen die bei $R^1$ genannten Alkylgruppen, Halogenalkylgruppen,

Alkoxygruppen, Halogenalkoxygruppen und/oder Alkylthiogruppen mit jeweils ein bis vier Kohlenstoffatomen;

X Sauerstoff oder Schwefel;

Y, Z Stickstoff oder eine Methingruppe =CH-;

$R^4$ Halogen wie bei $R^1$ genannt, insbesondere Fluor, Chlor und Brom,

Alkyl oder Alkoxy mit jeweils ein bis vier Kohlenstoffatomen, insbesondere ein bis drei Kohlenstoffatomen, welche ein- bis fünffach durch Halogen, insbesondere Fluor und Chlor und/oder einfach durch Alkoxy oder Alkylthio mit jeweils ein bis vier, insbesondere ein bis zwei Kohlenstoffatomen, substituiert sein können;

Alkenyl, Alkenyloxy, Alkinyl oder Alkinyloxy mit jeweils drei bis sechs, insbesondere drei bis fünf Kohlenstoffatomen welches ein- bis fünffach durch Halogen, insbesondere Fluor und Chlor und/oder einfach durch Alkoxy oder Alkylthio mit jeweils ein bis vier, insbesondere ein bis zwei Kohlenstoffatomen, substituiert sein können;

$C_1$-$C_4$-Alkylamino wie Methylamino, Ethylamino, Propylamino, 1-Methylethylamino, Butylamino, 1-Methylpropylamino, 2-Methylpropylamino und 1,1-Dimethylethylamino, insbesondere Methylamino und 1,1-Dimethylethylamino;

$C_2$-$C_8$-Dialkylamino wie Di-Methylamino, Di-Ethylamino, Di-Propylamino, Di-1-Methylethylamino, Di-Butylamino, Di-1-Methylpropylamino, Di-2-Methylpropylamino, Di-1,1-Dimethylethylamino, Ethylmethylamino, Propylmethylamino, 1-Methylethylmethylamino, Butylmethylamino, 1-Methylpropylmethylamino, 2-Methylpropylmethylamino, 1,1-Dimethylethylmethylamino, Propylethylamino, 1-Methylethylethylamino, Butylethylamino, 1-Methylpropylethylamino, 2-Methylpropylethylamino, 1,1-Dimethylethylethylamino, 1-Methylethylpropylamino, Butylpropylamino, 1-Methylpropylpropylamino, 2-Methylpropylpropylamino, 1,1-Dimethylethylpropylamino, 1-Methylethylbutylamino, 1-Methylpropylbutylamino, 2-Methylpropylbutylamino und 1,1-Dimethylethylbutylamino, insbesondere Dimethylamino, Diethylamino und Di-1-Methylethylamino;

eine Phenylaminogruppe oder eine N-Alkylphenylaminogruppe wie N-Methylphenylamino, N-Ethylphenylamino, N-Propylphenylamino, N-1-Methylethylphenylamino, N-Butylphenylamino, N-1-Methylpropylphenylamino, N-2-Methylpropylphenylamino und N-1,1-Dimethylethylphenylamino, insbesondere N-Methylphenylamino und N-1,1-Dimethylethylphenylamino;

$R^5$ Wasserstoff oder im allgemeinen und im besonderen einer der Reste $R^4$

mit der Maßgabe, daß $R^2$ eine $C_1$-$C_4$-Halogenalkylgruppe oder Y eine Methingruppe =CH- oder $R^4$ eine $C_3$-$C_6$-Alkenylgruppe, welche ein bis fünf Halogenatome tragen kann oder eine $C_3$-$C_6$-Halogenalkenyloxygruppe bedeutet, wenn $R^1$ für -$OR^6$ und X für Sauerstoff steht, sowie ferner mit der Maßgabe, daß wenn $R^1$ für $OR^6$ und X für Schwefel steht, $R^6$ eine $C_3$-$C_6$-Alkenyl- oder $C_3$-$C_6$-Alkinylgruppe darstellt, wobei die Gruppen 1 bis 5 Halogenatome tragen können, sowie deren umweltverträglichen Salze.

Insbesondere als herbizide Wirkstoffe bevorzugt sind Verbindungen der Formel I, in denen

$R^1$ einen Rest ON = $CR^7R^8$

bedeutet sowie solche, in denen

$R^1$ einen unsubstituierten oder substituierten 5-gliedrigen Heterocyclus bedeutet

und solche, in denen

X Schwefel,

Y Stickstoff und

Z eine Methingruppe bedeutet.

Im Hinblick auf die pflanzenwachstumsregulierende Wirkung sind Verbindungen Ia bevorzugt, in denen die Substituenten folgende Bedeutung haben:

$R^1$ Hydroxy,

$C_1$-$C_{10}$-Alkoxi, z.B. Methoxi, Ethoxi, Propoxi, Butyloxi, Pentyloxi oder Hexyloxi, das gegebenenfalls durch $C_1$-$C_3$-Alkylthio, $C_1$-$C_3$-Alkoxi, Phenoxi, $C_1$-$C_4$-Alkylcarbonyl oder Phenylcarbonyl substituiert ist,

Phenyl-($C_1$-$C_3$)-alkoxi, das unsubstituiert oder im Phenylteil ein- bis dreifach durch Halogen wie Fluor, Chlor, Brom und Jod, Methoxi und Methyl substituiert ist,

$C_3$-$C_6$-Alkenyloxi, das gegebenenfalls durch 1 bis 5 Halogen, insbesondere Chlor und Brom, substituiert ist,

niedermolekulares, z.B. $C_2$-$C_4$-Alkinyloxi, insbesondere Propargyloxi,

$C_3$-$C_{10}$-Alkoxicarbonylalkoxi, insbesondere $C_3$-$C_7$-Alkoxicarbonylalkoxy, z.B. Methoxicarbonylmethoxi, Ethoxicarbonylmethoxi, n-Propoxycarbonylmethoxi, Methoxicarbonylethoxi, Ethoxicarbonylethoxi und Ethoxicarbonylpropyloxi,

5-gliedriges Heteroaryl, wie insbesondere Imidazolyl, Pyrazolyl, 1,2,3- und 1,2,4-Triazolyl,

ein Rest ON = $CR^7R^8$ (Alkylidenaminoxi), welcher sich von symmetrischen oder unsymmetrischen, verzweigten oder unverzweigten $C_3$-$C_{20}$-, vorzugsweise $C_3$-$C_{15}$-, besonders bevorzugt $C_3$-$C_{11}$-(Alkylketonen) oder $C_8$-$C_{18}$-, vorzugsweise $C_8$-$C_{13}$-(Alkylphenylketonen) ableitet, gegebenenfalls durch ein- bis dreifach durch Methyl substituiertes $C_4$-$C_{12}$-, vorzugsweise $C_5$-$C_8$-Cycloalkylidenaminoxi,

$R^2$, $R^3$ $C_1$-$C_4$-Alkyl,
$C_1$-$C_4$-Halogenalkyl, z.B. mit 1 bis 4 Halogenatomen wie Fluor, Chlor und Brom, wobei $C_1$-$C_2$-Chlor- und Fluoralkyl wie beispielsweise Trifluormethyl und Difluormethyl, bevorzugt ist,
$C_1$-$C_4$-Alkoxi und Alkylthio, z.B. $C_1$-$C_3$-Alkoxi und $C_1$-$C_3$-Alkylthio,
$C_1$-$C_4$-Halogenalkyloxi, wobei die oben genannten Halogenalkylreste bevorzugt sind,

X Sauerstoff,

Y Stickstoff,

Z Stickstoff oder die Methingruppe =CH-,

$R^4$ Halogen wie Fluor, Chlor, Brom und Jod,
verzweigtes oder unverzweigtes $C_1$-$C_4$-Alkyl,
$C_1$-$C_4$-Halogenalkyl wie für $R^2$ und $R^3$ genannt,
unsubstituiertes oder durch $C_1$-$C_3$-Alkyl oder Halogen, wie Chlor und Fluor substituiertes Alkenyl, z.B. $C_2$-$C_6$-, insbesondere $C_2$-$C_4$-Alkenyl,
$C_1$-$C_4$-Alkoxi, unsubstituiertes oder durch Halogen wie Fluor, Chlor und Brom substituiertes $C_3$-$C_6$-Alkenyloxi,
$C_3$-$C_4$-Alkinyloxi, wie Propargyloxi, und

n 0, 1 oder 2.

Unter Alkyl oder Alkyl in einer Alkoxigruppe ist je nach der genannten Zahl der Kohlenstoffatome Methyl, Ethyl, Propyl, Isopropyl, Butyl, sek.-Butyl, tert.-Butyl, Pentyl und Isomere, Hexyl und Isomere, Heptyl und Isomere oder Octyl und Isomere zu verstehen. Entsprechend sind auch bei den höheren Homologen die Isomeren jeweils mit einbezogen. Cycloalkyl in der Cycloalkylidenaminogruppe bedeutet beispielsweise Cyclobutyl, Cyclopentyl, Cyclohexyl, Cycloheptyl oder Cyclooctyl. Alkenyl oder Alkenyl in einer Alkenyloxigruppe steht z.B. für Allyl, Propenyl, Butenyl, Buten-2-yl, Pentenyl, Hexenyl oder Heptenyl.

Von den vorstehenden Verbindungen sind einige besonders zu erwähnen, nämlich solche, bei denen

$R^1$ Hydroxi, unsubstituiertes oder durch $C_1$-$C_2$-Alkoxi oder $C_1$-$C_2$-Alkylthio substituiertes $C_1$-$C_5$-Alkoxi, Benzyloxi, $C_2$-$C_5$-Alkenyloxi, das unsubstituiert oder durch Methyl oder Chlor substituiert ist, Propargyloxi, Alkylidenaminoxi, das sich von verzweigten oder unverzweigten $C_3$-$C_{11}$-(Alkylketonen) ableitet, $C_5$-$C_8$-Cycloalkylidenaminoxi oder Imidazolyl,

$R^2$, $R^3$ Trifluormethyl, $C_1$-$C_3$-Alkoxi, Difluormethoxi oder $C_1$-$C_3$-Alkylthio,

Z Stickstoff oder die Methingruppe,

$R^4$ Fluor, Chlor, Brom, $C_1$-$C_4$-Alkyl, Allyl, Chlorallyl, $C_1$-$C_2$-Alkoxi, Allyloxi oder Chlorallyloxi bedeutet.

Als Salze der Verbindungen der Formel Ia kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I und Ia bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I und Ia eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung

von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I bzw. Ia können beispielsweise wie folgt formuliert werden:

I. Man vermischt 90 Gewichtsteile der Verbindung Nr. 1.001 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.008 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.011 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.047 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.022 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.014 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.010 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des wirkstoffs mit guter Haftfähigkeit.

VIII. 20 Gewichtsteile des Wirkstoffs Nr. 1.008 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 2.018 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 2.043 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

XI. 20 Gewichtsteile der Verbindung Nr. 2.013 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XII. 20 Gewichtsteile des Wirkstoffs Nr. 2.009 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XIII. 20 Gewichtsteile des Wirkstoffs Nr. 2.009 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

XIV. 3 Gewichtsteile des Wirkstoffs Nr. 2.013 werden mit 97 Gewichtsteilen feinteiligem Kaolin vermischt. Man erhält auf diese Weise ein Stäubemittel, das 3 Gew.% des Wirkstoffs enthält.

XV. 30 Gewichtsteile des Wirkstoffs Nr. 2.012 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 2.014 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an herbizidem Wirkstoff betragen je nach Bekämpfungsziel, Jahreszeit, Zielpflanzen und Wachstumsstadium 0,001 bis 3,0, vorzugsweise 0,005 bis 0,5 kg/ha aktive Substanz (a.S.).

Die Salicylsäurederivate der Formel Ia können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel Ia im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Salicylsäurederivate der Formel Ia Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden

- die Durchwurzelung des Bodens verbessert wird und
- das Mikroklima im Pflanzenbestand durch einen kompakteren Wuchs günstig beeinflußt wird.

Die erfindungsgemäß zu verwendenden Wirkstoffe der Formel Ia können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Plfanzenverträglichkeit kann die Aufwandmenge stark variiert werden.

Bei der Saatgutbehandlung werden im allgemeinen Wirkstoffmengen von 0,001 bis 50 g, vorzugsweise 0,01 bis 10 g, je Kilogramm Saatgut, benötigt.

Für die Blatt- und Bodenbehandlung sind im allgemeinen Gaben von 0,001 bis 10 kg/ha bevorzugt 0,01 bis 3 kg/ha als ausreichend zu betrachten.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen I und Ia mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone,

Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I und Ia allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung Von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I und Ia benutzt. Die so erhaltenen Verbindungen sind in den nachstehenden Tabellen mit physikalischen Angaben aufgeführt. Verbindungen ohne diese Angaben lassen sich aus den entsprechenden Edukten analog synthetisieren. Die in der Tabelle wiedergegebenen Strukturen beschreiben besonders bevorzugte Wirkstoffe der Formel I bzw. Ia.

Beispiel 1

Herstellung von Methyl-5-allyloxi-2-(4,6-dimethoxipyrimidin-2-yl)oxibenzoat:

Zu 3,3 g 4,6-Dimethoxi-2-methylsulfonylpyrimidin in 70 ml Ethylmethylketon gibt man 3,2 g Methyl-5-allyloxi-2-hydroxibenzoat in 10 ml Ethylmethylketon sowie 20 g Kaliumcarbonat. Es wird 4,5 h zum Sieden erhitzt, in Eiswasser gegossen und mit Methylenchlorid extrahiert. Nach dem Trocknen über Natriumsulfat wird eingeengt und das zurückbleibende Öl wird durch Chromatographie an Silica-Gel gereinigt. Schmelzpunkt: 74-75 °C.

Beispiel 2

Allgemeine Vorschrift zur Herstellung substituierter Benzoesäuren der Formel I:

5,1 g Kaliumhydroxid und 0,08 Mol der jeweiligen 2-Hydroxibenzoesäure werden in 80 ml Methanol gelöst, 10 min bei Raumtemperatur gerührt und im Vakuum eingeengt. Anschließend wird zum Trocknen wiederholt mit Toluol versetzt und dieses bei 50 °C im Vakuum verdampft. Das so erhaltene hellrote Pulver wird in 300 ml Dimethylsulfoxid aufgenommen und bei Raumtemperatur portionsweise mit 2,9 g 80 %igem Natriumhydrid versetzt wobei eine Gasentwicklung auftritt. Wenn kein Gas mehr frei wird, tropft man eine Lösung von 17,4 g 4,6-Dimethoxi-2-methylsulfonylpyrimidin in 80 ml Dimethylsulfoxid zu und rührt 30 Minuten nach. Man gießt in 2 l Wasser, neutralisiert mit Essigsäure und wäscht mit Methylenchlorid. Anschließend wird mit Salzsäure stark angesäuert und mehrmals mit Methyl-tert.-butylether extrahiert. Man trocknet über Natriumsulfat und verdampft das Lösungsmittel i.V. Die zurückbleibende Substanz kann durch Chromatographie an Silica-Gel gereinigt werden.

Beispiel 3

Allgemeine Vorschrift zur Herstellung substituierter Benzoesäureoximester oder ähnlicher Verbindungen:

3,2 mmol der jeweiligen 2-(4,6-Dimethoxypyrimidin-2-yl)oxibenzoesäure werden in 20 ml Dimethoxyethan vorgelegt und mit 3,2 mmol Natriumhydrid versetzt, wobei sofort Gasentwicklung beobachtet wird. Es wird 1 h bei Raumtemperatur nachgerührt, auf 0 °C gekühlt und mit 3,5 mmol Oxalylchlorid versetzt. Man rührt bei 0 °C 1 h nach und verdampft dann zur Entfernung des überschüssigen Oxalylchlorids etwa 30 % des Lösungsmittels im Vakuum. Man gibt nun 4,2 mmol des jeweiligen Oxims oder einer vergleichbaren Hydroxi-Verbindung gelöst in 10 ml Dimethoxiethan und anschließend 3,2 mmol Pyridin bei 0 °C zu und erwärmt innerhalb von 1 h auf Raumtemperatur. Man gießt in 120 ml kaltes Wasser, extrahiert mit Methylenchlorid, trocknet über Natriumsulfat und engt ein. Die zurückbleibende Substanz kann durch Chromatographie an Silica-Gel weiter gereinigt werden.

Beispiel 4

Herstellung von 2-Chloro-6-(4,6-dimethoxitriazin-2-yloxi)benzoesäure

15,5 g 2-Chlor-6-hydroxibenzoesäure werden in1 50 ml Dimethylformamid vorgelegt und bei 25 °C portionsweise mit 20,2 g Kalium-tert.-butylat versetzt. Anschließend werden 15,7 g 2-Chlor-4,6-dimethoxytriazin zugegeben. Man rührt 12 h bei 25 °C nach, gießt in kaltes Wasser und säuert mit Salzsäure an. Anschließend wird mit Essigsäureethylester extrahiert, über Natriumsulfat getrocknet und das Lösungsmittel im Vakuum entfernt. Man verrührt den Rückstand mit etwas Diethylether, filtriert ab und trocknet den Rückstand im Vakuum (Schm. 128-131 °C).

Tabelle 1

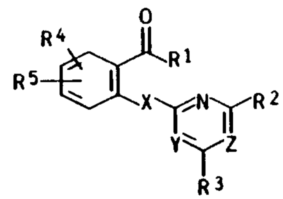

| Nr. | $R^1$ | $R^2$ | $R^3$ | $R^4$ | $R^5$ | X | Y | Z | phys.Dat.* |
|---|---|---|---|---|---|---|---|---|---|
| 1.001 | 2-Propaniminoxi | $OCH_3$ | $OCH_3$ | 6-Cl | H | O | N | CH | Fp.= 92 - 94°C |
| 1.002 | Cyclohexaniminoxi | $OCH_3$ | $OCH_3$ | 6-Cl | H | O | N | CH | δ=2,40(m); 3,80 (s); 5,75(s) |
| 1.003 | Cyclopentaniminoxi | $OCH_3$ | $OCH_3$ | 6-Cl | H | O | N | CH | |
| 1.004 | 1-Phenylethan-1-iminoxi | $OCH_3$ | $OCH_3$ | 6-Cl | H | O | N | CH | |
| 1.005 | Succinyliminoxi | $OCH_3$ | $OCH_3$ | 6-Cl | H | O | N | CH | |
| 1.006 | 1-Imidazolyl | $OCH_3$ | $OCH_3$ | 6-Cl | H | O | N | CH | Fp.= 87°C |
| 1.007 | $OCH_3$ | $OCH_3$ | $OCH_3$ | 6-Cl | H | S | N | CH | |
| 1.008 | OH | $OCH_3$ | $OCH_3$ | 6-Cl | H | S | N | CH | Fp.=156 - 159°C |
| 1.009 | OH | $OCH_3$ | $OCH_3$ | 6-Cl | H | S | N | N | |
| 1.010 | OH | $CF_3$ | $OCH_3$ | 6-Cl | H | S | N | CH | |
| 1.011 | $OCH_3$ | $OCH_3$ | $OCH_3$ | 3-Allyl | 6-Cl | O | N | CH | δ=3,28(d); 3,75(s); 3,80(s); 5,05(m) |
| 1.012 | OH | $OCH_3$ | $OCH_3$ | 3-Allyl | 6-Cl | O | N | CH | |
| 1.013 | $OCH_3$ | $OCH_3$ | $OCH_3$ | 3-(2-methyl-allyl) | 6-Cl | O | N | CH | |
| 1.014 | n-Decanoxi | $OCH_3$ | $OCH_3$ | 6-Cl | H | S | N | CH | |
| 1.015 | 2-Chlorallyloxi | $OCH_3$ | $OCH_3$ | 6-Cl | H | O | N | CH | |

| Nr. | $R^1$ | $R^2$ | $R^3$ | $R^4$ | $R^5$ | X | Y | Z | phys.Dat.* |
|---|---|---|---|---|---|---|---|---|---|
| 1.016 | 2-Propaniminoxi | $OCHF_2$ | $CH_3$ | 6-Cl | H | O | N | CH | |
| 1.017 | 2-Butaniminoxi | $OCH_3$ | $OCH_3$ | 6-Cl | H | S | N | CH | |
| 1.018 | Benzyloxi | $CH_3$ | $CH_3$ | 5-Cl | H | S | N | CH | |
| 1.019 | 3-Chlorbenzyloxi | $OCH_3$ | $OCH_3$ | 5-Cl | H | S | N | CH | |
| 1.020 | OH | $OCH_3$ | $OCH_3$ | 6-Cl | H | O | CH | N | |
| 1.021 | OH | $OCH_3$ | $CF_3$ | 6-Cl | H | O | CH | CH | |
| 1.022 | OH | $OCH_3$ | $CF_3$ | 6-Cl | H | S | CH | CH | |
| 1.023 | 2-Propaniminoxi | $OCH_3$ | $OCH_3$ | 3-Cl | 5-Cl | O | N | CH | |
| 1.024 | 2-Methylcyclohexaniminoxi | $OCH_3$ | $OCH_3$ | 6-Cl | H | O | N | CH | |
| 1.025 | 3-Dodecaniminoxi | $OCH_3$ | $OCH_3$ | 6-Cl | H | O | N | CH | δ=0,88(t); 1,08(t); 3,80(s); 5,75(s) |
| 1.026 | 2-Methylhexan-3-iminoxi | $OCH_3$ | $OCH_3$ | 5-Br | H | O | N | CH | |
| 1.027 | 1-Imidazolyl | $OCH_3$ | $OCH_3$ | 6-Ethoxi | H | O | N | CH | |
| 1.028 | 2-Propaniminoxi | $OCH_3$ | $OCH_3$ | 6-F | H | O | N | CH | |
| 1.029 | ONa | $OCH_3$ | $OCH_3$ | 6-Cl | H | S | N | CH | |
| 1.030 | Methylthiomethoxy | $OCH_3$ | $OCH_3$ | 6-Cl | H | S | N | CH | Fp.= 90°C |
| 1.031 | $OCH_3$ | $OCH_3$ | $OCH_3$ | 3-(2-Chlor-allyl | 6-Cl | O | N | CH | δ=3,60(s); 3,75(s); 3,80(s); 5,75(s) |
| 1.032 | 1-([1,2,4]-Triazolyl) | $OCH_3$ | $OCH_3$ | 6-Cl | H | O | N | CH | |
| 1.033 | OH | $CF_3$ | $OCH_3$ | 6-Cl | H | O | N | CH | |
| 1.034 | OH | $OCH_3$ | $CH_3$ | 6-Cl | H | S | N | CH | |
| 1.035 | 2-Propaniminoxi | $OCH_3$ | $CH_3$ | 6-Cl | H | O | N | CH | |
| 1.036 | 2-Propaniminoxi | $OCH_3$ | $OCH_3$ | 6-F | H | O | N | N | |
| 1.037 | Cyclohexaniminoxy | $OCH_3$ | $OCH_3$ | 6-F | H | O | N | CH | |
| 1.038 | $OCH_3$ | $OCH_3$ | $OCH_3$ | 3-Allyl | 4-OH | O | N | CH | F.= 88 - 89°C |
| 1.039 | $OC_2H_4SC_2H_5$ | $OCH_3$ | $OCH_3$ | 6-Cl | H | S | N | CH | δ=1,20(t); 3,70(s); 4,43(t); 5,73(s) |

| Nr. | $R^1$ | $R^2$ | $R^3$ | $R^4$ | $R^5$ | X | Y | Z | phys.Dat.* |
|---|---|---|---|---|---|---|---|---|---|
| 1.040 | $OCH_2C{\equiv}CH$ | $OCH_3$ | $OCH_3$ | 6-Cl | H | S | N | CH | F.= 96 - 98°C |
| 1.041 | 1-Imidazolyl | $OCH_3$ | $OCH_3$ | 6-Cl | H | S | N | CH | F.=102 - 103°C |
| 1.042 | 2-Methylhexan-3-iminoxi | $OCH_3$ | $OCH_3$ | 6-Cl | H | O | N | CH | δ=1,13(d); 3,80(s); 5,75(s) |

*Fp.: Schmelzpunkt

$n_D$: Brechungsindex,

δ: $^1$H-NMR - chemische Verschiebung in ppm (ausgewählte Signale).

Tabelle 2

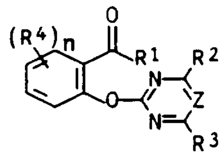

Ia

| Bsp. | $R^1$ | $R^2$ | $R^3$ | $(R^4)_n$ | Z | phys. Dat* |
|---|---|---|---|---|---|---|
| 2.001 | 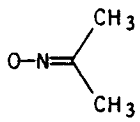 | $OCH_3$ | $OCH_3$ | 6-Cl | CH | 92- 94 |
| 2.002 | 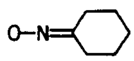 | $OCH_3$ | $OCH_3$ | 6-Cl | CH | |
| 2.003 | 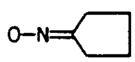 | $OCH_3$ | $OCH_3$ | 6-Cl | CH | |
| 2.004 | 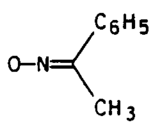 | $OCH_3$ | $OCH_3$ | 6-Cl | CH | |
| 2.007 | 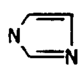 | $OCH_3$ | $OCH_3$ | 6-Cl | CH | 87 |
| 2.008 | $OCH_3$ | $OCH_3$ | $OCH_3$ | 6-$CH_3$ | N | 83- 85 |
| 2.009 | OH | $OCH_3$ | $OCH_3$ | 6-Cl | N | 128-131 |
| 2.010 | $OCH_3$ | $OCH_3$ | $OCH_3$ | 6-Cl | N | 71- 74 |
| 2.011 | $OCH_3$ | $SCH_3$ | $SCH_3$ | 6-Cl | N | 89- 90 |
| 2.012 | OH | $OCH_3$ | $OCH_3$ | 5-$OCH_2CH=CH_2$ | CH | 143-145 |
| 2.013 | $OCH_3$ | $OCH_3$ | $OCH_3$ | 5-$OCH_2CH=CH_2$ | CH | 74- 75 |
| 2.014 | $OCH_3$ | $OCH_3$ | $OCH_3$ | 6-$OCH_2CH=CH_2$ | CH | 84- 85 |

Tabelle 2 (Fortsetzung)

| Bsp. | $R^1$ | $R^2$ | $R^3$ | $(R^4)_n$ | Z | phys. Dat* |
|---|---|---|---|---|---|---|
| 2.015 | $OCH_3$ | $OCH_3$ | $OCH_3$ | $6\text{-}O\text{-}CH_2CH_2OCH_3$ | CH | 83 - 85 |
| 2.016 | $OCH_3$ | $OCH_3$ | $OCH_3$ | $5\text{-}OCH_2CH{=}CH_2$ | N | |
| 2.017 | $OCH_3$ | $SCH_3$ | $OCH_3$ | $5\text{-}OCH_2CH{=}CH_2$ | N | |
| 2.018 | $OCH_3$ | $OCH_3$ | $OCH_3$ | $3\text{-}CH_2CH{=}CH_2$, 6-Cl | CH | |
| 2.019 | OH | $OCH_3$ | $OCH_3$ | $3\text{-}CH_2CH{=}CH_2$, 6-Cl | CH | |
| 2.020 | $OCH_3$ | $OCH_3$ | $OCH_3$ | 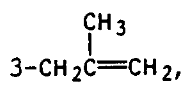, 6-Cl | CH | |
| 2.021 | $OCH_2C_6H_5$ | $OCH_3$ | $OCH_3$ | $5\text{-}OCH_2CH{=}CH_2$ | CH | |
| 2.022 | 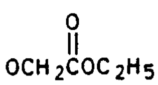 | $OCH_3$ | $OCH_3$ | 6-Cl | CH | |
| 2.023 | 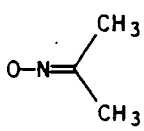 | $OCH_3$ | $OCH_3$ | $5\text{-}OCH_2CH{=}CH_2$ | CH | |
| 2.024 | $OCH_2CH{=}CH_2$ | $OCH_3$ | $OCH_3$ | 6-Cl | CH | |
| 2.025 | 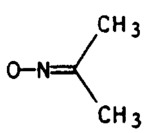 | $OCHF_2$ | $CH_3$ | 6-Cl | CH | |
| 2.026 | OH | $OCH_3$ | $SCH_3$ | 6-Cl | N | |
| 2.027 | $OCH_2C{\equiv}CH$ | $OCH_3$ | $OCH_3$ | 6-Cl | CH | |
| 2.028 | $OCH_2C{\equiv}CH$ | $OCH_3$ | $OCH_3$ | $5\text{-}OCH_2CH{=}CH_2$ | CH | |

Tabelle 2 (Fortsetzung)

| Bsp. | $R^1$ | $R^2$ | $R^3$ | $(R^4)_n$ | Z | phys. Dat* |
|---|---|---|---|---|---|---|
| 2.029 | $OCH_3$ | $OCH_3$ | $OCH_3$ | 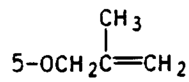 | CH | |
| 2.030 | $OCH_3$ | $OCH_3$ | $OCH_3$ | 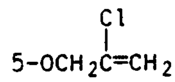 | CH | |
| 2.031 | 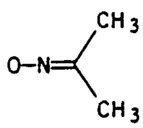 | $OCH_3$ | $OCH_3$ | 3-Cl, 5-Cl | CH | |
| 2.032 | 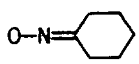 | $OCH_3$ | $OCH_3$ | $5-OCH_2CH=CH_2$ | CH | |
| 2.033 | 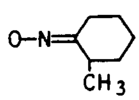 | $OCH_3$ | $OCH_3$ | 6-Cl | CH | |
| 2.034 | 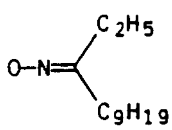 | $OCH_3$ | $OCH_3$ | 6-Cl | CH | |
| 2.035 | 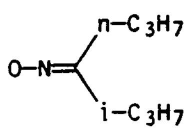 | $OCH_3$ | $OCH_3$ | 5-Br | CH | |
| 2.036 | $O-i-C_3H_7$ | $OCH_3$ | $OCH_3$ | $6-OCH_2CH=CH_2$ | CH | |
| 2.037 | 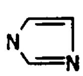 | $OCH_3$ | $OCH_3$ | $6-OC_2H_5$ | CH | |
| 2.038 | 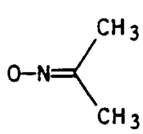 | $OCH_3$ | $OCH_3$ | 6-F | CH | |

Tabelle 2 (Fortsetzung)

| Bsp. | $R^1$ | $R^2$ | $R^3$ | $(R^4)_n$ | Z | phys. Dat* |
|---|---|---|---|---|---|---|
| 2.039 | 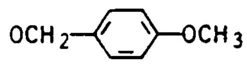 | $OCH_3$ | $OCH_3$ | 4-$CH_3$ | N | |
| 2.040 | $OCH_2OC_2H_5$ | $OCH_3$ | $OCH_3$ | 6-Cl | N | |
| 2.041 | OH | $OCH_3$ | $OCH_3$ | 6-F | N | |
| 2.042 | 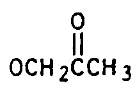 | $OCH_3$ | $OCH_3$ | 6-Cl | N | |
| 2.043 | $OCH_3$ | $OCH_3$ | $OCH_3$ | 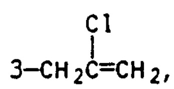, 6-Cl | CH | δ=3,60(s); 3,75(s); 3,80(s); 5,75(s) |
| 2.044 | 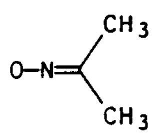 | $OCH_3$ | $OCH_3$ | 6-Cl | N | |

* Fp.: Schmelzpunkt in °C,
$n_D$: Brechungsindex,
δ: $^1$H-NMR - chemische Verschiebung in ppm (ausgewählte Signale), gemessen in $CDCl_3$.

Anwendungsbeispiele zur herbiziden Wirkung

Die herbizide Wirkung der Verbindungen der Formel I ließ sich durch Gewächshausversuche zeigen: Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird. Die Aufwandmengen betrugen 0,03 kg/ha.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmengen für die Nachauflaufbehandlung betrugen 0,03 und 0,06 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| ABUTH | Abutilon theophrasti | Chinesischer Hanf |
| BROIN | Bromus inermis | unbegrannte Trespe |
| CHEAL | Chenopodium album | Weißer Gänsefuß |
| ECHCG | Echinochloa crus-galli | Hühnerhirse |
| GALAP | Galium aparine | Klettenlabkraut |
| LAMAM | Lamium amplexicaule | Stengelumfassende Taubnessel |
| NICPH | Nicandra physaloides | - |
| SETIT | Setaria italica | Ital. Raygras |
| SOLNI | Solanum nigrum | Schwarzer Nachtschatten |

Mit 0,03 bzw. 0,06 kg/ha a.S. im Vor- bzw. Nachauflaufverfahren eingesetzt, lassen sich mit den Beispielen 1.001 und 1.002 unerwünschte Gräser bzw. breitblättrige unerwünschte Pflanzen sehr gut bekämpfen.

Anwendungsbeispiele zur wachstumsregulierenden Wirkung

Als Vergleichssubstanz "A" diente in den jeweiligen Beispielen 2-Chlorethyltrimethylammoniumchlorid (CCC):

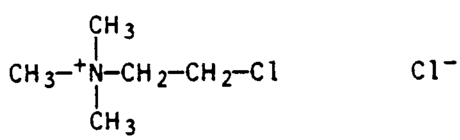

Beispiel I

Untersuchung auf wachstumsregulierende Wirkung in Zellsuspensionen von Mais (Grossmann und Jung 1984, Plant Cell Rep. 3, 156-158).

Das Verfahren sieht die Kultivierung von 2 ml Suspension in sterilen Plastikreagenzröhrchen vor. Die Testsubstanzen wurden in Aceton gelöst und der Kultur in einer Konzentration von $10^{-4}$ mol•$1^{-1}$ zugefügt. Nach 8 Tagen Inkubationszeit wurde die Leitfähigkeit des Kulturmediums als Wachstumsparameter bestimmt und die Hemmung des Wachstums in % zur Kontrolle verrechnet (0 = keine Hemmung, 100 = totale Hemmung des Wachstums).

Die Versuchsergebnisse zeigen, daß im Testsystem mit Zellsuspensionen die erfindungsgemäßen Mittel mit den Verbindungen 2.009, 2.012, 2.013, 2.018 und 2.043 als Wirkstoff effektiv das Wachstum der Zellen hemmen (66 bis 93 % Hemmung) und dem Vergleichsmittel A (14 % Hemmung) deutlich in der Wirkung überlegen sind.

Beispiel II

Untersuchung auf wachstumsregulierende Wirkung in einem Prüfsystem mit Wasserlinsen (Lemna paucicostata).

Die Pflanzen wurden photomixotroph (Zusatz von 1% Saccharose im anorganischen Nährmedium) unter sterilen Bedingungen im Dauerlicht angezogen. Die Prüfsubstanzen wurden-in Aceton gelöst und in

Aufwandmengen von $10^{-4}$ bis $10^{-8}$ mol/l den Wasserlinsen zugegeben. Nach 8 Tagen wurde die Frischgewichtszunahme der Pflanzen bestimmt und die wachstumsregulierende Wirkung der Verbindungen in % Hemmung des Wachstums zur Kontolle verrechnet (0 = keine Hemmung, 100 = totale Hemmung des Wachstums).

Die Versuchsergebnisse zeigen, daß die erfindungsgemäßen Mittel mit den Verbindungen 2.009, 2.012, 2.013, 2.014, 2.015 und 2.018 als aktive Substanz im Testsystem deutlich stärker das Wachstum der Wasserlinsen hemmen als das Vergleichsmittel A. So tritt z.B. bei einer molaren Konzentration von $10^{-4}$ eine Wachstumshemmung von 89 bis 96 % auf, während das Vergleichsmittel A nur 32 % Hemmung bewirkte.

Beispiel III

Zur Bestimmung der wachstumsregulierenden Eigenschaft der Prüfsubstanzen wurden die Testpflanzen auf ausreichend mit Nährstoffen versorgtem Kultursubstrat in Kunststoffgefäßen (ca. 12,5 cm Durchmesser; Volumen ca. 500 ml) angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzelheiten sind den Tabellen IIa, IIb und IIc zu entnehmen.

Tabelle IIa

| Sommerweizen, Sorte "Ralle" | | |
|---|---|---|
| Nr. der chem. Beispiele | Konz. mg WS/Gef.* | Wuchshöhen rel. |
| unbehandelt | - | 100 |
| A | 0,05<br>0,1<br>0,38<br>1,5 | 90,5<br>87,3<br>87,3<br>81,1 |
| 2.009 | 0,05<br>0,1<br>0,38<br>1,5 | 53,0<br>53,0<br>53,0<br>49,9 |
| 2.012 | 0,05<br>0,1<br>0,38<br>1,5 | 100,0<br>96,7<br>71,7<br>53,0 |
| 2.013 | 0,05<br>0,1<br>0,38<br>1,5 | 71,7<br>67,1<br>53,0<br>49,9 |

* Wirkstoff/Gefäß

Tabelle IIb

| Sommergerste, Sorte "Aramir" | | |
|---|---|---|
| Nr. der chem. Beispiele | Konz. mg WS/Gef.* | Wuchshöhen rel. |
| unbehandelt | - | 100 |
| A | 0,05<br>0,1<br>0,38<br>1,5 | 94,9<br>94,9<br>94,9<br>94,9 |
| 2.009 | 0,05<br>0,1<br>0,38<br>1,5 | 72,0<br>72,0<br>49,1<br>45,8 |
| 2.012 | 0,05<br>0,1<br>0,38<br>1,5 | 100,0<br>99,8<br>94,9<br>62,2 |
| 2.013 | 0,05<br>0,1<br>0,38<br>1,5 | 91,6<br>88,3<br>75,3<br>49,1 |

* Wirkstoff/Gefäß

Tabelle IIc

| Sonnenblume, Sorte "Spanners Allzweck" | | |
|---|---|---|
| Nr. der chem. Beispiele | Konz. mg WS/Gef.* | Wuchshöhen rel. |
| unbehandelt | - | 100 |
| A | 0,38<br>1,5 | 93,0<br>93,0 |
| 2.043 | 0,38<br>1,5 | 100<br>76,8 |

* Wirkstoff/Gefäß

## Patentansprüche

**Patentansprüche für folgende Vertragsstaaten : AT, BE, CH, DE, FR, GB, IT, LI, NL, SE**

**1.** Salicylsäurederivate und deren Schwefelanaloge der Formel I,

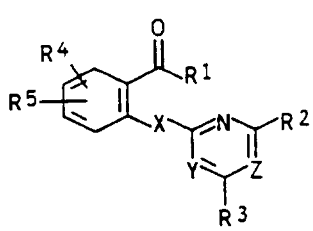

I

in der die Substituenten folgende Bedeutung haben:

$R^1$ eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest -$OR^6$, worin

$R^6$ Wasserstoff, ein Alkalimetallkation, das Äquivalent eines Erdalkalimetallkations oder ein organisches Ammoniumion;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, oder
eine $C_3$-$C_6$-Alkenyl- oder eine $C_3$-$C_6$-Alkinylgruppe, wobei diese Gruppen ihrerseits ein bis fünf Halogenatome tragen können;
bedeutet, oder
einen Rest $OH = CR^7R^8$, worin

$R^7$ und $R^8$ $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann
bedeutet;

$R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

X ein Sauerstoff- oder Schwefelatom;

Y,Z ein Stickstoffatom oder eine Methingruppe =CH-;

$R^4$ ein Halogenatom,
eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio;
eine $C_3$-$C_6$-Alkenyl-, $C_3$-$C_6$-Alkenyloxy-$_1$ $C_3$-$C_6$-Alkinyl- oder $C_3$-$C_6$-Alkinyloxygruppe, welche ein bis fünf Halogenatome und/oder einen der folgenden Reste tragen kann: $C_1$-$C_4$-Alkoxy oder $C_1$-$C_4$-Alkylthio;
eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe;

$R^5$ Wasserstoff oder einen der Reste $R^4$

mit der Maßgabe, daß $R^2$ eine $C_1$-$C_4$-Halogenalkylgruppe oder Y eine Methingruppe =CH- oder $R^4$ eine $C_3$-$C_6$-Alkenylgruppe, welche ein bis fünf Halogenatome tragen kann oder eine $C_3$-$C_6$-Halogenalkenyloxygruppe bedeutet, wenn $R^1$ für -$OR^6$ und X für Sauerstoff steht, sowie ferner mit der Maßgabe, daß wenn $R^1$ für $OR^6$ und X für Schwefel steht, $R^6$ eine $C_3$-$C_6$-Alkenyl- oder $C_3$-$C_6$-Alkinylgruppe darstellt, wobei die Gruppen 1 bis 5 Halogenatome tragen können, sowie deren umweltverträglichen Salze.

**2.** Verfahren zur Herstellung der Verbindungen der Formel I gemäß Anspruch 1, dadurch gekennzeichnet, daß man eine entsprechende ortho-Mercapto- oder ortho-Hydroxibenzoesäureverbindung der Formel II

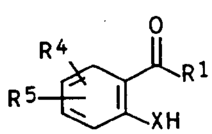

II

in an sich bekannter Weise mit einem Heterocyclus der Formel III,

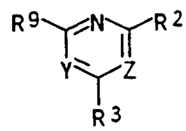

III

worin $R^9$ für eine nucleofuge Abgangsgruppe steht, in Gegenwart einer anorganischen oder organischen Base umsetzt.

**3.** Verfahren zur Herstellung der Verbindungen der Formel I gemäß Anspruch 1, dadurch gekennzeichnet, daß man die freie Salicylsäure bzw. ihre Schwefelanalogen der Formel I'

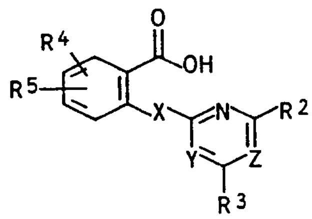

I'

zunächst in an sich bekannter Weise in das Halogenid oder eine andere aktivierte Form der Carbonsäure überführt und diese mit einem Oxim, N-Hydroxisuccinylimin oder einem Alkenylalkohol umsetzt.

**4.** Herbizides Mittel enthaltend eine Verbindung der Formel I gemäß Anspruch 1.

**5.** Herbizide Mittel gemäß Anspruch 4, enthaltend weitere wirksame Bestandteile.

**6.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

**7.** Verwendung von Salicylsäurederivaten und deren Schwefel analogen der Formel I gemäß Anspruch 1 als Herbizide.

**8.** Verwendung von Salicylsäurederivaten und deren Schwefelanalogen der allgemeinen Formel Ia

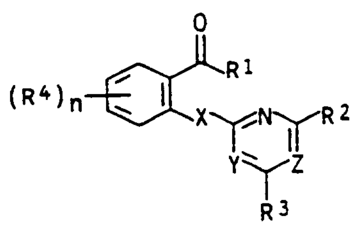

Ia

in der die Substituenten folgende Bedeutung haben:

$R^1$ eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest -$OR^6$, worin

$R^6$ Wasserstoff, ein Alkalimetallkation, das Äquivalent eines Erdalkalimetallkations oder ein organisches Ammoniumion;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, oder
eine $C_3$-$C_6$-Alkenyl- oder eine $C_3$-$C_6$-Alkinylgruppe, wobei diese Gruppen ihrerseits ein bis fünf Halogenatome tragen können;
bedeutet, oder
einen Rest $OH=CR^7R^8$, worin

$R^7$ und $R^8$ $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann bedeutet;

$R^2$, $R^3$ $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

X ein Sauerstoffatom;

Y ein Stickstoffatom;

Z ein Stickstoffatom oder eine Methingruppe =CH-;

$R^4$ ein Halogenatom,
eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio;
eine $C_3$-$C_6$-Alkenyl-, $C_3$-$C_6$-Alkenyloxy-, $C_3$-$C_6$-Alkinyl- oder $C_3$-$C_6$-Alkinyloxygruppe, welche ein bis fünf Halogenatome und/oder einen der folgenden Reste tragen kann: $C_1$-$C_4$-Alkoxy oder $C_1$-$C_4$-Alkylthio;
eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe;

n 0, 1 oder 2 bedeutet oder dessen pflanzenverträgliche Salze

zur Beeinflussung des Pflanzenwachstums.

9. Verfahren zur Regulierung des Pflanzenwachstums, dadurch gekennzeichnet, daß man eine regulativ wirksame Menge eines Salicylsäurederivats der allgemeinen Formel Ia gemäß Anspruch 8 auf die Samen, die Pflanzen und/oder deren Lebensraum einwirken läßt.

**Patentansprüche für folgenden Vertragsstaat : ES**

1. Verfahren zur Herstellung von Salicylsäurederivaten und deren Schwefelanalogen der Formel I,

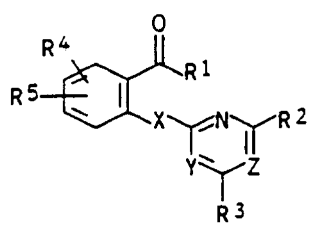

I

in denen die Substituenten folgende Bedeutung haben:

$R^1$ eine Succinyliminooxygruppe;
ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
einen Rest $-OR^6$, worin

$R^6$ Wasserstoff, ein Alkalimetallkation, das Äquivalent eines Erdalkalimetallkations oder ein organisches Ammoniumion;
eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Haloge-

nalkoxy und/oder $C_1$-$C_4$-Alkylthio, oder
eine $C_3$-$C_6$-Alkenyl- oder eine $C_3$-$C_6$-Alkinylgruppe, wobei diese Gruppen ihrerseits ein bis fünf Halogenatome tragen können;
bedeutet, oder
einen Rest $ON=CR^7R^8$, worin

| | |
|---|---|
| $R^7$ und $R^8$ | $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann bedeutet; |
| $R^2$, $R^3$ | $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio; |
| X | ein Sauerstoff- oder Schwefelatom; |
| Y,Z | ein Stickstoffatom oder eine Methingruppe =CH-; |
| $R^4$ | ein Halogenatom, eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio; eine $C_3$-$C_6$-Alkenyl-, $C_3$-$C_6$-Alkenyloxy-, $C_3$-$C_6$-Alkinyl- oder $C_3$-$C_6$-Alkinyloxygruppe, welche ein bis fünf Halogenatome und/oder einen der folgenden Reste tragen kann: $C_1$-$C_4$-Alkoxy oder $C_1$-$C_4$-Alkylthio; eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe; |
| $R^5$ | Wasserstoff oder einen der bei $R^4$ genannten Gruppen; |

sowie deren umweltverträglichen Salze, mit der Maßgabe, daß $R^2$ eine $C_1$-$C_4$-Halogenalkylgruppe oder Y eine Methingruppe =CH- oder $R^4$ eine $C_3$-$C_6$-Alkenylgruppe, welche ein bis fünf Halogenatome tragen kann oder eine $C_3$-$C_6$-Halogenalkenyloxygruppe bedeutet, wenn $R^1$ für $-OR^6$ und X für Sauerstoff steht, sowie ferner mit der Maßgabe, daß wenn $R^1$ für $OR^6$ und X für Schwefel steht, $R^6$ eine $C_3$-$C_6$-Alkenyl- oder $C_3$-$C_6$-Alkinylgruppe darstellt, wobei die Gruppen 1 bis 5 Halogenatome tragen können, dadurch gekennzeichnet, daß man eine entsprechende ortho-Mercapto- oder ortho-Hydroxibenzoesäureverbindung der Formel II

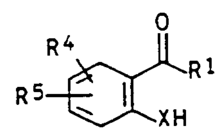

II

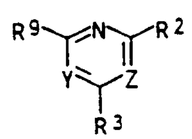

III

worin $R^9$ für eine nucleofuge Abgangsgruppe steht, in Gegenwart einer anorganischen oder organischen Base umsetzt.

2. Verfahren zur Herstellung der Verbindungen der Formel I gemäß Anspruch 1, dadurch gekennzeichnet, daß man die freie Salicylsäure bzw. ihre Schwefelanalogen der Formel I'

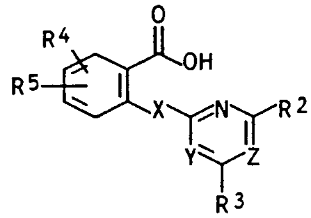

I'

zunächst in an sich bekannter Weise in das Halogenid oder eine andere aktivierte Form der Carbonsäure überführt und diese mit einem Oxim, N-Hydroxisuccinylimin oder einem Alkenylalkohol umsetzt.

**3.** Herbizides Mittel enthaltend eine Verbindung der Formel I gemäß Anspruch 1.

**4.** Herbizide Mittel gemäß Anspruch 3, enthaltend weitere wirksame Bestandteile.

**5.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

**6.** Verwendung von Salicylsäurederivaten und deren Schwefelanalogen der Formel I gemäß Anspruch 1 als Herbizide.

**7.** Verwendung von Salicylsäurederivaten und deren Schwefelanalogen der allgemeinen Formel Ia

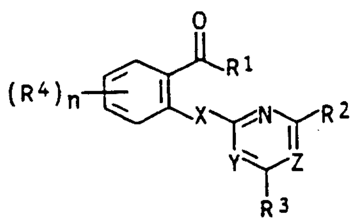

Ia

in der die Substituenten folgende Bedeutung haben:

| | |
|---|---|
| $R^1$ | eine Succinyliminooxygruppe; |
| | ein 5-gliedriger Heteroaromat, enthaltend ein bis drei Stickstoffatome, welcher ein bis vier Halogenatome und/oder ein bis zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio; |
| | einen Rest $-OR^6$, worin |
| $R^6$ | Wasserstoff, ein Alkalimetallkation, das Äquivalent eines Erdalkalimetallkations oder ein organisches Ammoniumion; |
| | eine $C_1$-$C_{10}$-Alkylgruppe welche ein bis fünf Halogenatome und/oder einen der folgenden Reste tragen kann: $C_1$-$C_4$-Alkoxy, $C_1$-$C_4$-Alkylthio, Cyano, $C_1$-$C_8$-Alkylcarbonyl, $C_1$-$C_8$-Alkoxycarbonyl, Phenyl, Phenoxy oder Phenylcarbonyl, wobei die aromatischen Reste ihrerseits ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio, oder |
| | eine $C_3$-$C_6$-Alkenyl- oder eine $C_3$-$C_6$-Alkinylgruppe, wobei diese Gruppen ihrerseits ein bis fünf Halogenatome tragen können; |
| | bedeutet, oder |
| | einen Rest $ON=CR^7R^8$, worin |
| $R^7$ und $R^8$ | $C_1$-$C_{20}$-Alkyl, welches einen Phenylrest tragen kann, Phenyl oder gemeinsam eine $C_3$-$C_{12}$-Alkylenkette, welche ein bis drei $C_1$-$C_3$-Alkylgruppen tragen kann |
| | bedeutet; |
| $R^2$, $R^3$ | $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio; |
| X | ein Sauerstoffatom; |
| Y | ein Stickstoffatom; |
| Z | ein Stickstoffatom oder eine Methingruppe =CH-; |
| $R^4$ | ein Halogenatom, |
| | eine $C_1$-$C_4$-Alkyl- oder $C_1$-$C_4$-Alkoxygruppe, welche ein bis fünf Halogenatome und/oder eine der folgenden Gruppen tragen kann: $C_1$-$C_4$-Alkoxy oder $C_1$-$C_4$-Alkylthio; |
| | eine $C_3$-$C_6$-Alkenyl-, $C_3$-$C_6$-Alkenyloxy-, $C_3$-$C_6$-Alkinyl- oder $C_3$-$C_6$-Alkinyloxygruppe, welche ein bis fünf Halogenatome und/oder einen der folgenden Reste tragen kann: |

$C_1$-$C_4$-Alkoxy oder $C_1$-$C_4$-Alkylthio;
eine $C_1$-$C_4$-Alkylaminogruppe, eine $C_2$-$C_8$-Dialkylaminogruppe, eine Phenylaminogruppe oder eine N-$C_1$-$C_4$-Alkyl-N-phenylaminogruppe und
n 0, 1 oder 2 bedeutet oder dessen pflanzenverträgliche Salze
zur Beeinflussung des Pflanzenwachstums.

**8.** Verfahren zur Regulierung des Pflanzenwachstums, dadurch gekennzeichnet, daß man eine regulativ wirksame Menge eines Salicylsäurederivats der allgemeinen Formel Ia gemäß Anspruch 7 auf die Samen, die Pflanzen und/oder deren Lebensraum einwirken läßt.

## Claims

**Claims for the following Contracting States : AT, BE, CH, DE, FR, GB, IT, LI, NL, SE**

**1.** A salicylic acid derivative or a sulfur analog thereof of the formula I

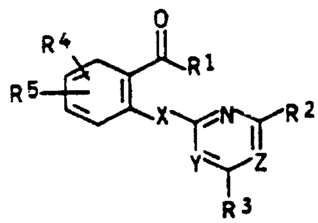

I

where $R^1$ is a succinyliminooxy group;
a 5-membered heteroaromatic ring which contains from one to three nitrogen atoms and may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
a radical -$OR^6$, where
$R^6$ is hydrogen, an alkali metal cation, the equivalent of an alkaline earth metal cation or an organic ammonium ion;
$C_1$-$C_{10}$-alkyl which may carry from one to five halogen atoms and/or one of the following radicals: $C_1$-$C_4$-alkoxy, $C_1$-$C_4$-alkylthio, cyano, $C_1$-$C_8$-alkylcarbonyl, $C_1$-$C_8$-alkoxycarbonyl, phenyl, phenoxy or phenylcarbonyl, where the aromatic radicals in turn may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio, or
$C_3$-$C_6$-alkenyl or $C_3$-$C_6$-alkynyl, where these groups in turn may carry from one to five halogen atoms;
or a radical $ON=CR^7R^8$, where
$R^7$ and $R^8$ are each $C_1$-$C_{20}$-alkyl which may carry a phenyl radical or are each phenyl or together form a $C_3$-$C_{12}$-alkylene chain which may carry from one to three $C_1$-$C_3$-alkyl groups;
$R^2$ and $R^3$ are each $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
X is oxygen or sulfur;
Y and Z are each nitrogen or a methine group =CH-;
$R^4$ is halogen,
a $C_1$-$C_4$-alkyl or $C_1$-$C_4$-alkoxy group which may carry from one to five halogen atoms and/or one of the following groups: $C_1$-$C_4$-alkoxy or $C_1$-$C_4$-alkylthio;
a $C_3$-$C_6$-alkenyl, $C_3$-$C_6$-alkenyloxy, $C_3$-$C_6$-alkynyl or $C_3$-$C_6$-alkynyloxy group which may carry from one to five halogen atoms and/or one of the following radicals: $C_1$-$C_4$-alkoxy or $C_1$-$C_4$-alkylthio;
$C_1$-$C_4$-alkylamino, $C_2$-$C_8$-dialkylamino, phenylamino or N-$C_1$-$C_4$-alkyl-N-phenylamino;
$R^5$ is hydrogen or one of the radicals $R^4$ with the proviso that $R^2$ is $C_1$-$C_4$-haloalkyl or Y is a methine group =CH- or $R^4$ is $C_3$-$C_6$-alkenyl, which may carry from one to five halogen atoms, or is $C_3$-$C_6$-haloalkenyloxy when R' is -$OR^6$ and X is oxygen, and with the further proviso that when $R^1$ is $OR^6$ and X is sulfur, $R^6$ is $C_3$-$C_6$-alkenyl or $C_3$-$C_6$-alkynyl, where these groups may carry from 1 to 5 halogen atoms, and their environmentally compatible salts.

**2.** A process for the preparation of a compound of the formula I as claimed in claim 1, wherein a corresponding ortho-mercapto or ortho-hydroxybenzoic acid compound of the formula II

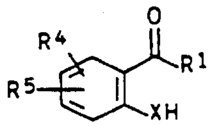

II

is reacted in a conventional manner with a heterocycle of the formula III

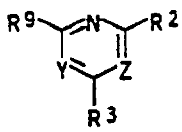

III

where $R^9$ is a nucleofugic leaving group, in the presence of an inorganic or organic base.

**3.** A process for the preparation of a compound of the formula I as claimed in claim 1, wherein the free salicylic acid or its sulfur analogs of the formula I'

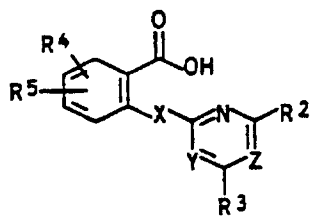

I'

is first converted in a conventional manner into the halide or another activated form of the carboxylic acid, and this is reacted with an oxime, N-hydroxysuccinylimine or an alkenyl alcohol.

**4.** A herbicidal agent containing a compound of the formula I as claimed in claim 1.

**5.** A herbicidal agent as claimed in claim 4, containing further active constituents.

**6.** A method for controlling undesirable plant growth, wherein the undesirable plants and/or their habitat are or is treated with a herbicidally effective amount of a derivative I as claimed in claim 1.

**7.** The use of a salicylic acid derivative or a sulfur analog thereof of the formula I as claimed in claim 1 as a herbicide.

**8.** The use of a salicylic acid derivative or a sulfur analog thereof of the general formula Ia

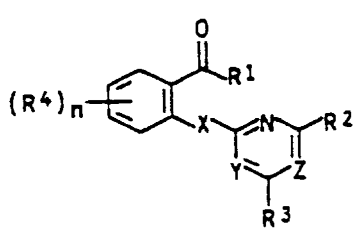

Ia

where
$R^1$ is a succinyliminooxy group;
a 5-membered heteroaromatic ring which contains from one to three nitrogen atoms and may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
a radical $-OR^6$, where

$R^6$ is hydrogen, an alkali metal cation, the equivalent of an alkaline earth metal cation or an organic ammonium ion;

$C_1$-$C_{10}$-alkyl which may carry from one to five halogen atoms and/or one of the following radicals: $C_1$-$C_4$-alkoxy, $C_1$-$C_4$-alkylthio, cyano, $C_1$-$C_8$-alkylcarbonyl, $C_1$-$C_8$-alkoxycarbonyl, phenyl, phenoxy or phenylcarbonyl, where the aromatic radicals in turn may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio, or

$C_3$-$C_6$-alkenyl or $C_3$-$C_6$-alkynyl, where these groups in turn may carry from one to five halogen atoms; or

a radical $ON=CR^7R^8$, where

$R^7$ and $R^8$ are each $C_1$-$C_{20}$-alkyl which may carry a phenyl radical or are each phenyl or together form a $C_3$-$C_{12}$-alkylene chain which may carry from one to three $C_1$-$C_3$-alkyl groups;

$R^2$ and $R^3$ are each $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;

X is oxygen;

Y is nitrogen;

Z is nitrogen or a methine group $=CH-$;

$R^4$ is halogen,

a $C_1$-$C_4$-alkyl or $C_1$-$C_4$-alkoxy group which may carry from one to five halogen atoms and/or one of the following groups: $C_1$-$C_4$-alkoxy or $C_1$-$C_4$-alkylthio;

a $C_3$-$C_6$-alkenyl, $C_3$-$C_6$-alkenyloxy, $C_3$-$C_6$-alkynyl or $C_3$-$C_6$-alkynyloxy group which may carry from one to five halogen atoms and/or one of the following radicals: $C_1$-$C_4$-alkoxy or $C_1$-$C_4$-alkylthio;

$C_1$-$C_4$-alkylamino, $C_2$-$C_8$-dialkylamino, phenylamino or N-$C_1$-$C_4$-alkyl-N-phenylamino; and

n is 0, 1 or 2,

or a plant-compatible salt thereof, for influencing plant growth.

**9.** A method for regulating plant growth, wherein a regulating amount of a salicylic acid derivative of the general formula Ia as claimed in claim 8 is allowed to act on the seed, the plants and/or their habitat.

**Claims for the following Contracting State : ES**

**1.** A process for the preparation of a salicylic acid derivative or a sulfur analog thereof of the formula I

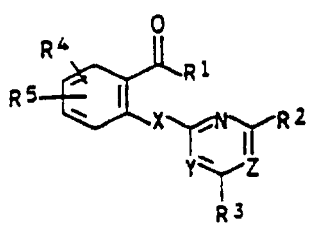

I

where $R^1$ is a succinyliminooxy group;

a 5-membered heteroaromatic ring which contains from one to three nitrogen atoms and may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;

a radical $-OR^6$, where

$R^6$ is hydrogen, an alkali metal cation, the equivalent of an alkaline earth metal cation or an organic ammonium ion;

$C_1$-$C_{10}$-alkyl which may carry from one to five halogen atoms and/or one of the following radicals: $C_1$-$C_4$-alkoxy, $C_1$-$C_4$-alkylthio, cyano, $C_1$-$C_8$-alkylcarbonyl, $C_1$-$C_8$-alkoxycarbonyl, phenyl, phenoxy or phenylcarbonyl, where the aromatic radicals in turn may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio, or

$C_3$-$C_6$-alkenyl or $C_3$-$C_6$-alkynyl, where these groups in turn may carry from one to five halogen atoms; or a radical $ON=CR^7R^8$, where

$R^7$ and $R^8$ are each $C_1$-$C_{20}$-alkyl which may carry a phenyl radical or are each phenyl or together form

a $C_3$-$C_{12}$-alkylene chain which may carry from one to three $C_1$-$C_3$-alkyl groups;
$R^2$ and $R^3$ are each $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
X is oxygen or sulfur;
Y and Z are each nitrogen or a methine group =CH-;
$R^4$ is halogen,
a $C_1$-$C_4$-alkyl or $C_1$-$C_4$-alkoxy group which may carry from one to five halogen atoms and/or one of the following groups: $C_1$-$C_4$-alkoxy or $C_1$-$C_4$-alkylthio;
a $C_3$-$C_6$-alkenyl, $C_3$-$C_6$-alkenyloxy, $C_3$-$C_6$-alkynyl or $C_3$-$C_6$-alkynyloxy group which may carry from one to five halogen atoms and/or one of the following radicals: $C_1$-$C_4$-alkoxy or $C_1$-$C_4$-alkylthio;
$C_1$-$C_4$-alkylamino, $C_2$-$C_8$-dialkylamino, phenylamino or N-$C_1$-$C_4$-alkyl-N-phenylamino;
$R^5$ is hydrogen or one of the groups mentioned for $R^4$;
or an environmentally compatible salt thereof, with the proviso that $R^2$ is $C_1$-$C_4$-haloalkyl or Y is a methine group =CH- or $R^4$ is $C_3$-$C_6$-alkenyl, which may carry from one to five halogen atoms, or is $C_3$-$C_6$-haloalkenyloxy when $R^1$ is -$OR^6$ and X is oxygen, and with the further proviso that when R1 is $OR^6$ and X is sulfur, $R^6$ is $C_3$-$C_6$-alkyl or $C_3$-$C_6$-alkynyl, where these groups may carry from 1 to 5 halogen atoms, and their environmentally compatible salts, wherein a corresponding ortho-mercapto or ortho-hydroxybenzoic acid compound of the formula II

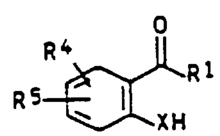

II

is reacted in a conventional manner with a heterocycle of the formula III

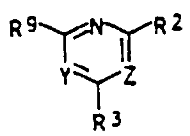

III

where $R^9$ is a nucleofugic leaving group, in the presence of an inorganic or organic base.

**2.** A process for the preparation of a compound of the formula I as claimed in claim 1, wherein the free salicylic acid or its sulfur analogs of the formula I'

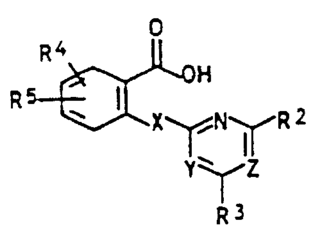

I'

is first converted in a conventional manner into the halide or another activated form of the carboxylic acid, and this is reacted with an oxime, N-hydroxysuccinylimine or an alkenyl alcohol.

**3.** A herbicidal agent containing a compound of the formula I as claimed in claim 1.

**4.** A herbicidal agent as claimed in claim 3, containing further active constituents.

**5.** A method for controlling undesirable plant growth, wherein the undesirable plants and/or their habitat are or is treated with a herbicidally effective amount of a derivative I as claimed in claim 1.

6. The use of a salicylic acid derivative or a sulfur analog thereof of the formula I as claimed in claim 1 as a herbicide.

7. The use of a salicylic acid derivative or a sulfur analog thereof of the general formula Ia

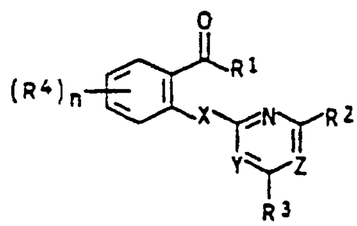

Ia

where
$R^1$ is a succinyliminooxy group;
a 5-membered heteroaromatic ring which contains from one to three nitrogen atoms and may carry from one to four halogen atoms and/or one or two of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
a radical -$OR^6$, where
$R^6$ is hydrogen, an alkali metal cation, the equivalent of an alkaline earth metal cation or an organic ammonium ion;
$C_1$-$C_{10}$-alkyl which may carry from one to five halogen atoms and/or one of the following radicals: $C_1$-$C_4$-alkoxy, $C_1$-$C_4$-alkylthio, cyano, $C_1$-$C_8$-alkylcarbonyl, $C_1$-$C_8$-alkoxycarbonyl, phenyl, phenoxy or phenylcarbonyl, where the aromatic radicals in turn may carry from one to five halogen atoms and/or from one to three of the following radicals: $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio, or
$C_3$-$C_6$-alkenyl or $C_3$-$C_6$-alkynyl, where these groups in turn may carry from one to five halogen atoms; or
a radical $ON=CR^7R^8$, where
$R^7$ and $R^8$ are each $C_1$-$C_{20}$-alkyl which may carry a phenyl radical or are each phenyl or together form a $C_3$-$C_{12}$-alkylene chain which may carry from one to three $C_1$-$C_3$-alkyl groups;
$R^2$ and $R^3$ are each $C_1$-$C_4$-alkyl, $C_1$-$C_4$-haloalkyl, $C_1$-$C_4$-alkoxy, $C_1$-$C_4$-haloalkoxy and/or $C_1$-$C_4$-alkylthio;
X is oxygen;
Y is nitrogen;
Z is nitrogen or a methine group $=CH-$;
$R^4$ is halogen,
a $C_1$-$C_4$-alkyl or $C_1$-$C_4$-alkoxy group which may carry from one to five halogen atoms and/or one of the following groups: $C_1$-$C_4$-alkoxy or $C_1$-$C_4$-alkylthio;
a $C_3$-$C_6$-alkenyl, $C_3$-$C_6$-alkenyloxy, $C_3$-$C_6$-alkynyl or $C_3$-$C_6$-alkynyloxy group which may carry from one to five halogen atoms and/or one of the following radicals: $C_1$-$C_4$-alkoxy or $C_1$-$C_4$-alkylthio;
$C_1$-$C_4$-alkylamino, $C_2$-$C_8$-dialkylamino, phenylamino or N-$C_1$-$C_4$-alkyl-N-phenylamino; and
n is 0, 1 or 2,
or a plant-compatible salt thereof, for influencing plant growth.

8. A method for regulating plant growth, wherein a regulating amount of a salicylic acid derivative of the general formula Ia as claimed in claim 7 is allowed to act on the seeds, the plants and/or their habitat.

**Revendications**

**Revendications pour les Etats contractants suivants : AT, BE, CH, DE, FR, GB, IT, LI, NL, SE**

**1.** Dérivés de l'acide salicylique et leurs analogues sufurés de formule I

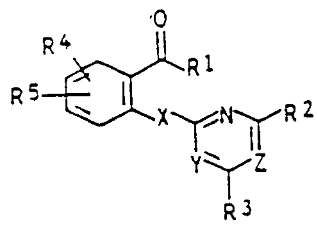

I

dans laquelle les symboles ont les significations suivantes :

$R^1$ représente un groupe succinyliminooxy ;
un cycle hétéroaromatique à 5 chaînons contenant 1 à 3 atomes d'azote et qui peut porter 1 à 4 atomes d'halogènes et/ou 1 à 2 des substituants suivants : alkyle en $C_1$-$C_4$,halogénoalkyle en $C_1$-$C_4$, alcoxy en $C_1$-$C_4$, halogénoalcoxy en $C_1$-$C_4$, alkylthio en $C_1$-$C_4$ ;
un groupe -$OR^6$ dans lequel

$R^6$ représente l'hydrogène, un cation d'un métal alcalin, l'équivalent d'un cation de métal alcalino-terreux ou un ion ammonium organique ;
un groupe alkyle en $C_1$-$C_{10}$ qui peut porter 1 à 5 atomes d'halogènes et/ou un des substituants suivants : alcoxy en $C_1$-$C_4$, alkylthio en $C_1$-$C_4$, cyano, alkylcarbonyle en $C_1$-$C_8$, (alcoxy en $C_1$-$C_8$)-carbonyle, phényle, phénoxy ou phénylcarbonyle, les radicaux aromatiques pouvant euxmêmes porter de 1 à 5 atomes d'halogènes et/ou de 1 à 3 des substituants suivants : alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$, ou bien
un groupe alcényle en $C_3$-$C_6$ ou alcynyle en $C_3$-$C_6$, ces groupes pouvant eux-mêmes porter de 1 à 5 atomes d'halogènes ;
ou bien
un groupe $ON=CR^7R^8$ dans lequel

$R^7$ et $R^8$ représentent chacun un groupe alkyle en $C_1$-$C_{20}$, qui peut porter un groupe phényle, un groupe phényle, ou bien $R^7$ et $R^8$ représentent ensemble une chaîne alkylène en $C_3$-$C_{12}$ qui peut porter 1 à 3 groupes alkyle en $C_1$-$C_3$ ;

$R^2$ et $R^3$ représentent chacun un groupe alkyle en $C_1$-$C_4$,halogénoalkyle en $C_1$-$C_4$, alcoxy en $C_1$-$C_4$, halogénoalcoxy en $C_1$-$C_4$ et/ou alkylthio en $C_1$-$C_4$ ;

X représente un atome d'oxygène ou de soufre,

Y, Z représentent chacun un atome d'azote ou le groupe méthine =CH- ;

$R^4$ représente un atome d'halogène,
un groupe alkyle en $C_1$-$C_4$ ou alcoxy en $C_1$-$C_4$ qui peut porter 1 à 5 atomes d'halogènes et/ou un des substituants suivants : alcoxy en $C_1$-$C_4$ ou alkylthio en $C_1$-$C_4$ ;
un groupe alcényle en $C_3$-$C_6$, alcényloxy en $C_3$-$C_6$, alcynyle en $C_3$-$C_6$ ou alcynyloxy en $C_3$-$C_6$, qui peut porter 1 à 5 atomes d'halogènes et/ou un des substituants suivants : alcoxy en $C_1$-$C_4$ ou alkylthio en $C_1$-$C_4$ ;
un groupe alkylamino en $C_1$-$C_4$, un groupe dialkylamino en $C_2$-$C_8$, un groupe phénylamino ou un groupe N-(alkyle en $C_1$-$C_4$)-N-phénylamino ;

$R^5$ représente l'hydrogène ou a l'une des significations de $R^4$,
sous réserve que $R^2$ représente un groupe halogénoalkyle en $C_1$-$C_4$ ou bien Y un groupe méthine =CH- ou $R_4$ un groupe alcényle en $C_3$-$C_6$ qui peut porter 1 à 5 atomes d'halogènes ou un groupe halogénoalcényloxy en $C_3$-$C_6$ lorsque $R^1$ représente - $OR^6$ et X un atome d'oxygène, et sous réserve également que, lorsque $R^1$ représente $OR^6$ et X le soufre, $R^6$ représente un groupe alcényle en $C_3$-$C_6$ ou alcynyle en $C_3$-$C_6$, ces groupes pouvant porter 1 à 5 atomes d'halogènes, et leurs sels non polluants.

2. Procédé de préparation des composés de formule I de la revendication 1, caractérisé en ce que l'on fait réagir un dérivé ortho-mercapto- ou ortho-hydroxy-benzoique correspondant de formule II

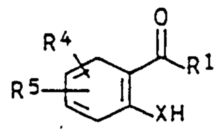

II

de manière connue en soi, avec un composé hétérocyclique de formule III

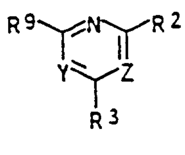

III

dans laquelle $R^9$ représente un groupe éliminable nucléofuge, en présence d'une base minérale ou organique.

3. Procédé de préparation des composés de formule I de la revendication 1, caractérisé en ce que l'on convertit d'abord, de manière connue en soi, l'acide salicyclique libre ou son analogue sulfuré libre de formule I'

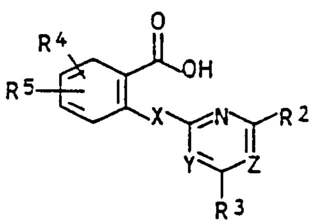

I'

en un halogénure ou en une autre forme activée de l'acide carboxylique qu'on fait réagir avec un oxime, la N-hydroxysuccinylimine ou un alcénol.

4. Produit herbicide contenant un composé de formule I de la revendication 1.

5. Produits herbicides selon la revendication 4, contenant d'autres composants actifs.

6. Procédé pour combattre les croissances de végétaux indésirables, caractérisé en ce que l'on traite les végétaux indésirables et/ou leur habitat par une quantité herbicide efficace d'un dérivé I de la revendication 1.

7. Utilisation des dérivés d'acide salicyclique et de leurs analogues sulfurés de formule I de la revendication 1 en tant qu'herbicides.

**8.** Utilisation des dérivés d'acide salicylique et de leurs analogues sulfurés de formule générale Ia

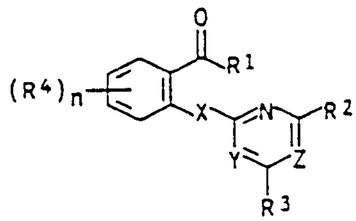

Ia

dans laquelle les symboles ont les significations suivantes :

$R^1$ représente un groupe succinyliminooxy ; un cycle hétéroaromatique de 5 chaînons contenant 1 à 3 atomes d'azote et qui peut porter 1 à 4 atomes d'halogènes et/ou 1 à 2 des substituants suivants : alkyle en $C_1$-$C_4$,halogénoalkyle en $C_1$-$C_4$, alcoxy en $C_1$-$C_4$, halogénoalcoxy en $C_1$-$C_4$ et/ou alkylthio en $C_1$-$C_4$ ;
un groupe -$OR^6$ dans lequel

$R^6$ représente l'hydrogène, un cation de métal alcalin, l'équivalent d'un cation de métal alcalino-terreux ou un ion ammonium organique ;
un groupe alkyle en $C_1$-$C_{10}$ qui peut porter 1 à 5 atomes d'halogènes et/ou un des substituants suivants : alcoxy en $C_1$-$C_4$, cyano, (alkyle en $C_1$-$C_8$)-carbonyle, (alcoxy en $C_1$-$C_8$)-carbonyle, phényle, phénoxy ou phénylcarbonyle, les radicaux aromatiques pouvant eux-mêmes porter de 1 à 5 atomes d'halogènes et/ou de 1 à 3 des substituants suivants : alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ; ou bien
un groupe alcényle en $C_3$-$C_6$ ou alcynyle en $C_3$-$C_6$, qui peut porter lui-même de 1 à 5 atomes d'halogènes ;
ou bien
un groupe $ON=CR^7R^8$ dans lequel

$R^7$ et $R^8$ représentent chacun un groupe alkyle en $C_1$-$C_{20}$ qui peut porter un groupe phényle, un groupe phényle, ou bien $R^7$ et $R^8$ représentent ensemble une chaîne alkylène en $C_3$-$C_{12}$ qui peut porter 1 à 3 groupes alkyle en $C_1$-$C_3$ ;

$R^2$ et $R^3$ représentent chacun un groupe alkyle en $C_1$-$C_4$,halogénoalkyle en $C_1$-$C_4$, alcoxy en $C_1$-$C_4$-halogénoalcoxy en $C_1$-$C_4$ et/ou alkylthio en $C_1$-$C_4$ ;

X représente un atome d'oxygène ;

Y représente un atome d'azote ;

Z représente un atome d'azote ou un groupe méthine =CH- ;

$R^4$ représente un atome d'halogène,
un groupe alkyle en $C_1$-$C_4$ ou alcoxy en $C_1$-$C_4$ qui peut porter 1 à 5 atomes d'halogènes et/ou un des substituants suivants : alcoxy en $C_1$-$C_4$ ou alkylthio en $C_1$-$C_4$ ;
un groupe alcényle en $C_3$-$C_6$, alcényloxy en $C_3$-$C_6$, alcynyle en $C_3$-$C_6$ ou alcynyloxy en $C_3$-$C_6$, qui peut porter 1 à 5 atomes d'halogènes et/ou un des substituants suivants : alcoxy en $C_1$-$C_4$ ou alkylthio en $C_1$-$C_4$ ;
un groupe alkylamino en $C_1$-$C_4$, un groupe dialkylamino en $C_2$-$C_8$, un groupe phénylamino ou un groupe N-(alkyle en $C_1$-$C_4$)-N-phénylamino ;

n est égal à 0, 1 ou 2,

ou de leurs sels tolérés par les végétaux, pour agir sur la croissance des végétaux.

**9.** Procédé pour la régulation de la croissance des végétaux, caractérisé en ce que l'on fait agir sur les semences, les végétaux et/ou leur habitat, une quantité régulatrice efficace d'un dérivé de l'acide salicylique de formule générale Ia de la revendication 8.

**Revendications pour l'Etat contractant suivant : ES**

**1.** Procédé de préparation de dérivés de l'acide salicylique et leurs analogues sufurés de formule I

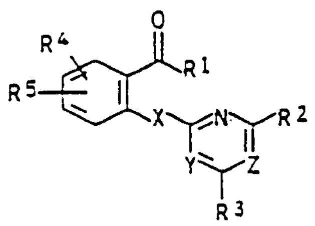

I

dans laquelle les symboles ont les significations suivantes :

$R^1$ représente un groupe succinyliminooxy ;
un cycle hétéroaromatique à 5 chaînons contenant 1 à 3 atomes d'azote et qui peut porter 1 à 4 atomes d'halogènes et/ou 1 à 2 des substituants suivants : alkyle en $C_1$-$C_4$,halogénoalkyle en $C_1$-$C_4$, alcoxy en $C_1$-$C_4$, halogénoalcoxy en $C_1$-$C_4$, alkylthio en $C_1$-$C_4$ ;
un groupe -$OR^6$ dans lequel

$R^6$ représente l'hydrogène, un cation d'un métal alcalin, l'équivalent d'un cation de métal alcalino-terreux ou un ion ammonium organique ;
un groupe alkyle en $C_1$-$C_{10}$ qui peut porter 1 à 5 atomes d'halogènes et/ou un des substituants suivants : alcoxy en $C_1$-$C_4$, alkylthio en $C_1$-$C_4$, cyano, (alkyle en $C_1$-$C_8$)-carbonyle, (alcoxy en $C_1$-$C_8$)-carbonyle, phényle, phénoxy ou phénylcarbonyle, les radicaux aromatiques pouvant euxmêmes porter de 1 à 5 atomes d'halogènes et/ou de 1 à 3 des substituants suivants : alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$, ou bien
un groupe alcényle en $C_3$-$C_6$, un groupe alcynyle en $C_3$-$C_6$, qui peut lui-mêmes porter de 1 à 5 atomes d'halogènes ;
ou bien
un groupe $ON=CR^7R^8$ dans lequel

$R^7$ et $R^8$ représentent chacun un groupe alkyle en $C_1$-$C_{20}$, qui peut porter un groupe phényle, un groupe phényle, ou bien $R^7$ et $R^8$ représentent ensemble une chaîne alkylène en $C_3$-$C_{12}$ qui peut porter 1 à 3 groupes alkyle en $C_1$-$C_3$ ;

$R^2$ et $R^3$ représentent chacun un groupe alkyle en $C_1$-$C_4$,halogénoalkyle en $C_1$-$C_4$, alcoxy en $C_1$-$C_4$, halogénoalcoxy en $C_1$-$C_4$ et/ou alkylthio en $C_1$-$C_4$ ;

X représente un atome d'oxygène ou le soufre,

Y, Z représentent chacun un atome d'azote ou le groupe méthine =CH- ;

$R^4$ représente un atome d'halogène,
un groupe alkyle en $C_1$-$C_4$ ou alcoxy en $C_1$-$C_4$ qui peut porter 1 à 5 atomes d'halogènes et/ou un des substituants suivants : alcoxy en $C_1$-$C_4$ ou alkylthio en $C_1$-$C_4$ ;
un groupe alcényle en $C_3$-$C_6$, alcényloxy en $C_3$-$C_6$, alcynyle en $C_3$-$C_6$ ou alcynyloxy en $C_3$-$C_6$, qui peut porter 1 à 5 atomes d'halogènes et/ou un des substituants suivants : alcoxy en $C_1$-$C_4$ ou alkylthio en $C_1$-$C_4$ ;
un groupe alkylamino en $C_1$-$C_4$, un groupe dialkylamino en $C_2$-$C_8$, un groupe phénylamino ou un groupe N-(alkyle en $C_1$-$C_4$)-N-phénylamino ;

$R^5$ représente l'hydrogène ou a l'une des significations de $R^4$,
sous réserve que $R^2$ représente un groupe halogénoalkyle en $C_1$-$C_4$ ou bien Y un groupe méthine =CH- ou $R_4$ un groupe alcényle en $C_3$-$C_6$ qui peut porter 1 à 5 atomes d'halogènes ou un groupe halogénoalcényloxy en $C_3$-$C_6$ lorsque $R^1$ représente - $OR^6$ et X un atome d'oxygène, et sous réserve également que, lorsque $R^1$ représente $OR^6$ et X le soufre, $R^6$ représente un groupe alcényle en $C_3$-$C_6$ ou alcynyle en $C_3$-$C_6$, ces groupes pouvant porter 1 à 5 atomes d'halogènes, caractérisé en ce que l'on fait réagir un dérivé correspondant ortho-mercapto- ou ortho-hydroxy-benzoique de formule II

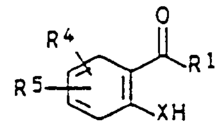

II

avec un composé hétérocyclique de formule III

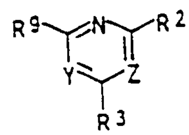

III

dans laquelle $R^9$ représente un groupe éliminable nucléofuge, en présence d'une base minérale ou organique.

2. Procédé de préparation des composés de formule I de la revendication 1, caractérisé en ce que l'on convertit d'abord de manière connue en soi l'acide salicylique libre ou son analogue sulfuré de formule I'

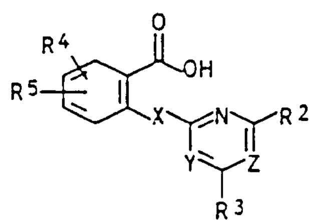

I'

en un halogénure ou en une autre forme activée de l'acide carboxylique qu'on fait réagir avec un oxime, la N-hydroxysuccinylimine ou un alcénol.

3. Produit herbicide contenant un composé de formule de la revendication 1.

4. Produits herbicides selon la revendication 3, contenant d'autres composants actifs.

5. Procédé pour combattre les croissances de végétaux indésirables, caractérisé en ce que l'on traite les végétaux indésirables et/ou leur habitat par une quantité herbicide efficace d'un dérivé I de la revendication 1.

6. Utilisation des dérivés d'acide salicylique et de leurs analogues sulfurés de formule I de la revendication 1, en tant qu'herbicides.

7. Utilisation des dérivés d'acide salicylique et de leurs analogues sulfurés de formule générale Ia

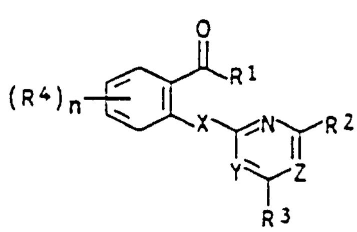

Ia

dans laquelle les symboles ont les significations suivantes :

$R^1$ représente un groupe succinyliminooxy ;
un cycle hétéroaromatique de 5 chaînons contenant 1 à 3 atomes d'azote et qui peut porter 1 à 4 atomes d'halogènes et/ou 1 à 2 des substituants suivants : alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ;
un groupe $-OR^6$ dans lequel

$R^6$ représente l'hydrogène, un cation de métal alcalin, l'équivalent d'un cation de métal alcalino-terreux ou un ion ammonium organique ;
un groupe alkyle en $C_1$-$C_{10}$ qui peut porter 1 à 5 atomes d'halogènes et/ou un des substituants suivants : alcoxy en $C_1$-$C_4$, cyano, (alkyle en $C_1$-$C_8$)-carbonyle, (alcoxy en $C_1$-$C_8$)-carbonyle, phényle, phénoxy ou phénylcarbonyle, les radicaux aromatiques pouvant eux-mêmes porter de 1 à 5 atomes d'halogènes et/ou de 1 à 3 des substituants suivants : alkyle en $C_1$-$C_4$, halogénoalkyle en $C_1$-$C_4$, alcoxy en $C_1$-$C_4$, halogénoalcoxy en $C_1$-$C_4$ et/ou alkylthio en $C_1$-$C_4$ ; ou bien
un groupe alcényle en $C_3$-$C_6$ ou alcynyle en $C_3$-$C_6$, qui peut porter lui-même de 1 à 5 atomes d'halogènes ;
ou bien
un groupe $ON=CR^7R^8$ dans lequel

$R^7$ et $R^8$ représentent chacun un groupe alkyle en $C_1$-$C_{20}$ qui peut porter un groupe phényle, un groupe phényle, ou bien $R^7$ et $R^8$ représentent ensemble une chaîne alkylène en $C_3$-$C_{12}$ qui peut porter 1 à 3 groupes alkyle en $C_1$-$C_3$ ;

$R^2$ et $R^3$ représentent chacun un groupe alkyle en $C_1$-$C_4$,halogénoalkyle en $C_1$-$C_4$, alcoxy en $C_1$-$C_4$-halogénoalcoxy en $C_1$-$C_4$ et/ou alkylthio en $C_1$-$C_4$ ;

X représente un atome d'oxygène ;

Y représente un atome d'azote ;

Z représente un atome d'azote ou un groupe méthine =CH- ;

$R^4$ représente un atome d'halogène,
un groupe alkyle en $C_1$-$C_4$ ou alcoxy en $C_1$-$C_4$ qui peut porter 1 à 5 atomes d'halogènes et/ou un des substituants suivants : alcoxy en $C_1$-$C_4$ ou alkylthio en $C_1$-$C_4$ ;
un groupe alcényle en $C_3$-$C_6$, alcényloxy en $C_3$-$C_6$, alcynyle en $C_3$-$C_6$ ou alcynyloxy en $C_3$-$C_6$, qui peut porter 1 à 5 atomes d'halogènes et/ou un des substituants suivants : alcoxy en $C_1$-$C_4$ ou alkylthio en $C_1$-$C_4$ ;
un groupe alkylamino en $C_1$-$C_4$, un groupe dialkylamino en $C_2$-$C_8$, un groupe phénylamino ou un groupe N-(alkyle en $C_1$-$C_4$)-N-phénylamino ;

n est égal à 0, 1 ou 2,

ou de leurs sels tolérés par les végétaux, pour agir sur la croissance des végétaux.

**8.** Procédé pour la régulation de la croissance des végétaux, caractérisé en ce que l'on fait agir sur les semences, les végétaux et/ou leur habitat, une quantité régulatrice efficace d'un dérivé de l'acide salicylique de formule générale Ia de la revendication 7.